(19) Europäisches Patentamt
European Patent Office
Office européen des brevets

(11) Publication number: **0 553 820 A2**

(12) **EUROPEAN PATENT APPLICATION**

(21) Application number: **93101321.3**

(22) Date of filing: **28.01.93**

(51) Int. Cl.[5]: **G01N 33/72**, C12Q 1/26, //G01N33/52

(30) Priority: **30.01.92 JP 38393/92**

(43) Date of publication of application: **04.08.93 Bulletin 93/31**

(84) Designated Contracting States: **DE ES FR GB IT**

(71) Applicant: **EIKEN KAGAKU KABUSHIKI KAISHA**
**33-8, Hongo 1-chome**
**Bunkyo-ku, Tokyo 114(JP)**

(72) Inventor: **Sato, Junji, c/o Eiken Kagaku Kabushiki Kaisha**
**143, Nogi, Nogi-machi, Shimotsuga-gun**
**Tochigi(JP)**
Inventor: **Kunii, Hitoshi, c/o Eiken Kagaku Kabushiki Kaisha**
**143, Nogi, Nogi-machi, Shimotsuga-gun**
**Tochigi(JP)**
Inventor: **Wada, Hiroshi, c/o Eiken Kagaku Kabushiki Kaisha**
**143, Nogi, Nogi-machi, Shimotsuga-gun**
**Tochigi(JP)**
Inventor: **Kosaka, Yuzo, c/o Eiken Kagaku Kabushiki Kaisha**
**143, Nogi, Nogi-machi, Shimotsuga-gun**
**Tochigi(JP)**

(74) Representative: **KUHNEN, WACKER & PARTNER**
**Alois-Steinecker-Strasse 22 Postfach 1553**
**W-8050 Freising (DE)**

(54) **Composition and test strip for measuring peroxidatively active substances.**

(57) A stable composition for use in the redox reaction-based measurement of peroxidatively active substances in a test sample (e.g., urine and blood), which can be produced easily, is stable for a prolonged period of time and does not cause false negatives due to the presence of a reducing substance in the test sample or false positives due to the addition of an oxidizing agent in the composition, as well as a test strip comprising a carrier incorporated with the composition. The composition includes a buffer material, a chromogen in an amount sufficient to react with the peroxidatively active substance in a test sample to develop color, a phosphonic acid analog of complexane as a stabilizing agent in an amount sufficient to stabilize the composition, and an oxidizing agent in an amount sufficient to oxidize reducing substances in a test sample.

EP 0 553 820 A2

## FIELD OF THE INVENTION

The present invention relates to a reagent composition for use in the detection of peroxidatively active substances (e.g., hemoglobin, myoglobin, myeloperoxidase, lactoperoxidase, etc.) in biological samples such as body fluids (e.g., blood), excrements (e.g., urine) and the like, and to a test strip prepared by making use of the composition. More particularly, it relates to a stable composition for use in the redox reaction-based measurement of peroxidatively active substances, which can be produced easily, is stable for a prolonged period of time and is free from the effect of reducing substances such as ascorbic acid and the like in test samples, and also to a test strip comprising a carrier incorporated with the composition is applied.

## BACKGROUND OF THE INVENTION

Detection of peroxidatively active substances such as blood (occult blood), contained in body fluids and excrements such as urine, vomit, gastrointestinal contents, feces and the like is exceedingly important from a clinical point of view for the early stage discovery of various diseases such as nephritis, tumors, angiostaxis, cystitis and the like. In many cases, however, blood volume in test samples such as excrements collected from patients of these diseases is extremely small and cannot be detected by the human eye.

In consequence, redox reaction-based measurement of peroxidatively active substances is widely used in the field of clinical chemistry, with a number of quick assay methods available. Especially, a means generally called "dry chemistry", such as a test strip method in which a reagent composition is supported on an absorbable carrier, has been used widely in recent years because of simple and quick handling for urine analysis.

In a typical example of the redox reaction-based method, occult blood in a test sample (e.g., urine) is measured by decomposing hydroperoxide making use of the peroxidase-like catalytic activity of hemoglobin contained in the test sample and subsequently effecting oxidation and coloring of a chromogen through its redox reaction with oxygen molecules generated by the hydroperoxide decomposition. Another example of the redox reaction-based method is measurement of glucose in test samples (e.g., urine and blood) based on a glucose oxidase/peroxidase/redox reaction system.

Thus, redox reaction is widely used in the field of clinical chemistry. In the test strip method, however, test samples are sometimes read as false positives, because hydroperoxide and a chromogen are both included in the same piece of test strip and these two ingredients gradually react with each other during a prolonged period of storage, thereby causing self-coloring of the test strip. In order to prevent such a phenomenon, attempts have been made to stabilize test strips by preventing direct contact between hydroperoxide and a chromogen making use of a separation means. For example, microcapsules are used in JP-B-39-14747 corresponding to U.S. Patent No. 3,252,762 (the term "JP-B" as used herein means an "examined Japanese patent publication"), a nylon mesh is used in JP-A-51-40980 corresponding to U.S. Patent No. 4,061,468 (the term "JP-A" as used herein means an "unexamined published Japanese patent application"), a film-forming polymer is used in JP-A-51-65694 corresponding to U.S. Patent No. 4,017,261 and a multiple layer structure is used in JP-A-3-242539. These prior art means, however, have a disadvantage in that the process of preparing these test strips becomes complex.

Other types of attempts for the prevention of self-coloring caused by the reaction of hydroperoxide with a chromogen have also been disclosed in which a stabilizing agent is used. For example, storage stability of test strips is effected by the addition of a phosphoric amide in the case of JP-A-49-54093 corresponding to U.S. Patent No. 3,853,471, acrylamide in the case of JP-A-53-143396 and JP-A-57-131061, a sulfoxide compound in the case of JP-A-53-115288 corresponding to U.S. Patent No. 4,071,318, ethylenediaminetetrakis(methylenephosphonic acid) in the case of JP-A-55-138655 and triethanolamine borate in the case of JP-A-57-103054 corresponding to U.S. Patent No. 4,071,318. Though long-term storage stability of test strips was improved by the addition of these stabilizing agents, such stabilizers do not avoid the effect of reducing substances in a test sample. That is, urine, blood and the like to be used as test samples contain reducing substances such as ascorbic acid and the like in many cases, and these reducing substances consume peroxidatively active substances thereby interfering with the results of redox reaction measurement, such as the generation of false negatives. The stabilizer-adding means, however, cannot avoid such influences of reducing substances.

With the aim of eliminating unwanted influences of reducing substances, a number of attempts have been made to use appropriate oxidizing agents. For example, JP-A-56-151358 corresponding to U.S. Patent Nos. 4,743,559 and 4,957,872 and JP-A-60-86467 corresponding to EP-A-141,224 disclose the use of

halogen oxyacids such as iodic acid, periodic acid and the like. Also, JP-A-57-13357 corresponding to U.S. Patent No. 4,310,626, JP-A-57-161650, JP-A-59-193354 corresponding to U.S. Patent No. 4,587,220, JP-A-62-169053 corresponding to U.S. Patent No. 4,755,472 and JP-A-3-30697 corresponding to U.S. Patent No. 4,954,451 disclose the use of metals such as cobalt (II, III), iron (III), cerium (IV) and the like in the form of complexes. The present applicants have also disclosed the use of an iodoxybenzene derivative as an oxidizing agent (JP-A-3-282259). Though these oxidizing agents are effective for the elimination of reducing substances in the test sample, the oxidizing agent itself oxidizes the chromogen when they are contained in the same layer of a carrier, thereby causing undesirable self-coloring prior to the use of test strips or resulting in false positives at the time of testing by developing a false color in spite of the absence of peroxidatively active substances.

## SUMMARY OF THE INVENTION

In view of the above, it therefore becomes an object of the present invention to provide a composition for use in the measurement of peroxidatively active substances in a test sample, which can be produced easily, is stable for a prolonged period of storage and does not cause false negatives due to the presence of a reducing substance in the sample or false positives due to the addition of an oxidizing agent in the composition, and also to provide a test strip comprising a carrier incorporated with the composition.

More particularly, the present invention provides a composition for use in the redox reaction-based measurement of peroxidatively active substances in a test sample, which comprises a buffer material, a chromogen in an amount sufficient to react with the peroxidatively active substance in a test sample to develop color, a phosphonic acid analog of complexane as a stabilizing agent in an amount sufficient to stabilize the composition, and an oxidizing agent in an amount sufficient to oxidize reducing substances in a test sample, as well as a test strip comprising a carrier incorporated with the composition.

Other objects and advantages of the present invention will be made apparent as the description progresses.

## DETAILED DESCRIPTION OF THE INVENTION

The stabilizing agent used in the present invention may preferably be selected from the group consisting of phosphonic acid analog compounds of complexane represented by the following formulae I, II and III:

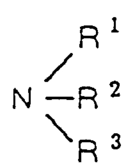

(I)

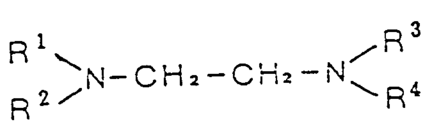

(II)

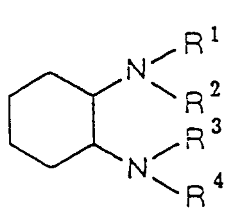

(III)

wherein $R^1$, $R^2$, $R^3$ and $R^4$ are the same or different groups, provided that at least one of the groups is an alkylenephosphonic acid group or a salt thereof, and the rest are selected from a hydrogen atom, a carboxyalkyl group and a hydroxyalkyl group.

The alkylenephosphonic acid group may preferably have from 1 to 5 carbon atoms and, more preferably, from 1 to 3 carbon atoms. The alkyl moiety of the carboxyalkyl group and the hydroxyalkyl group may preferably have from 1 to 5 carbon atoms and, more preferably, from 1 to 3 carbon atoms, respectively. Examples of the salt of the alkylenephosphonic acid group include the alkaline metal salts, the alkaline earth metal salts, the ammonium salt, the quaternary ammonium salts, etc. As the quaternary ammonium salts, alkyl-substituted ammonium salts such as $C_1$-$C_6$ alkyl-substituted ammonium salts may be mentioned.

The oxidizing agent may be selected preferably from the group consisting of iodoxybenzene compounds represented by the following formula IV, iodosobenzene compounds represented by the following formula V and halogen oxyacid compounds represented by the following formulae VI and VII:

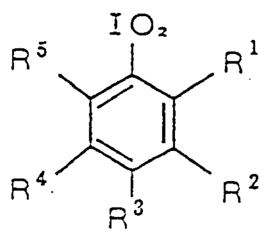

(IV) and

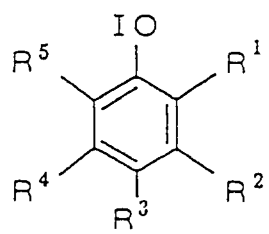

(V),

wherein $R^1$, $R^2$, $R^3$, $R^4$ and $R^5$ are the same or different groups selected from the class consisting of a hydrogen atom, a halogen atom, a carboxyl group, a sulfo group, a nitro group, a cyano group, a hydroxy group, an iodoxy group, a boro group, an arso group, a phospho group, and substituted or unsubstituted alkyl, alkoxy, aryl, arylsulfonyl, aryloxy, arylazo and amino groups; and

$MXO_3$ (VI)and

$MXO_4$ (VII)

wherein M is an atom or a group which is capable of becoming a monovalent cation, and X is a halogen atom.

Examples of the substituent of the alkyl, alkoxy, aryl, arylsulfonyl, aryloxy and arylazo groups include hydroxyl, cyano, nitro, halogeno, etc. and examples of the substituent of the amino group include alkyl etc. The alkyl and alkoxy groups may preferably have from 1 to 5 carbon atoms and, more preferably, from 1 to 3 carbon atoms, respectively. Each of the aryl group and the aryl moiety of the arylsulfonyl, aryloxy and arylazo groups may preferably be an aromatic group having from 6 to 10 ring atoms (C, N, S, O, etc.), more preferably, phenyl, tolyl, pyridyl, naphthyl, and quinolyl. Examples of M include a hydrogen atom, alkaline metals, ammonium, quaternary ammonium (e.g., alkyl-substituted ammonium such as $C_1$-$C_6$ alkyl-substituted ammonium), etc.

The buffer material and chromogen are not particularly limited, provided that they are compatible with a redox reaction. For example, when the reaction pH is in the range of from 4 to 8, citric acid, phosphoric acid, succinic acid, phthalic acid and salts thereof (e.g., alkaline metal salts, ammonium salts, etc.) may be used as the buffer material, and o-tolidine, o-dianisidine, 3,3',5,5'-tetramethylbenzidine and derivatives thereof may be used as the chromogen. The buffer material may be used preferably in an amount of from 0.01 mol/l to 1.5 mol/l, more preferably from 0.1 mol/l to 1.0 mol/l, as a concentration in an impregnating solution. The chromogen may be used preferably in an amount of from 1 mmol/l to 200 mmol/l, more preferably from 10 mmol/l to 100 mmol/l, as a concentration in an impregnating solution.

Types, properties and the like of phosphonic acid analogs of complexane to be added as a stabilizing agent are described in detail in *Kagaku–no Ryoiki*, vol.26, pp.581 - 583, 1972, the disclosure of which is incorporated by reference.

Preferred examples of the phosphonic acid analog of complexane represented by formula I include nitrilotris(methylenephosphonic acid), nitrilotris(ethylenephosphonic acid), nitrilotris(n-propylenephosphonic acid), nitrilodiacetic acid-methylenephosphonic acid, nitriloacetic acid-bis(methylenephosphonic acid), diethanolamine-N-methylenephosphonic acid), ethanolamine-N,N'-bis(methylenephosphonic acid) and the like.

Preferred examples of the analog represented by formula II include ethylenediaminetetrakis-(methylenephosphonic acid), ethylenediaminetetrakis(ethylenephosphonic acid), ethylenediaminetetrakis(n-propylelephosphonic acid), ethylenediamine-N,N'-bis(methylenephosphonic acid), ethylenediamine-N,N'-diacetic acid-N,N'-bis(methylenephosphonic acid), ethylenediamine-N,N'-bis(hydroxymethyl)-N,N'-bis-(methylenephosphonic acid) and the like.

Preferred examples of the analog represented by formula III include cyclohexane-1,2-diaminetetrakis-(methylenephosphonic acid), cyclohexane-1,2-diaminetetrakis(ethylenephosphonic acid), cyclohexane-1,2-diaminetetrakis(n-propylenephosphonic acid), cyclohexane-1,2-diamine-N,N'-diacetic acid-N,N'-bis-(methylenephosphonic acid), cyclohexane-1,2-diamine-N,N'-bis(hydroxymethyl)-N,N'-bis-(methylenephosphonic acid) and the like.

Of these analogs, particularly preferred are nitrilotris(methylenephosphonic acid), ethylenediaminetetrakis(methylenephosphonic acid) and cyclohexane-1,2-diaminetetrakis-(methylenephosphonic acid). The stabilizing agent may be used preferably in an amount of from 5 mmol/l to 130 mmol/l, more preferably from 10 mmol/l to 40 mmol/l, as a concentration in an impregnating solution.

Examples of the oxidizing agent represented by formula IV include iodoxybenzene, 3-iodoxybenzenecarboxylic acid, 4-iodoxybenzenecarboxylic acid, iodoxybenzene-3,4-dicarboxylic acid, 3-iodoxybenzenesulfonic acid, 4-iodoxybenzenesulfonic acid, 3-iodoxyphenol, 3-iodoxybenzenearsonic acid, 3-iodoxybenzeneboric acid and the like.

Examples of the oxidizing agent represented by formula V include iodosobenzene, 3-iodosobenzenecarboxylic acid, 3-iodosobenzenesulfonic acid, 3-iodosophenol, 3-iodosobenzenearsonic acid, 3-iodosobenzeneboric acid and the like.

Examples of the oxidizing agent represented by formula VI include iodic acid, potassium iodate, sodium iodate, bromic acid, potassium bromate, sodium bromate and the like.

Examples of the oxidizing agent represented by formula VII include periodic acid, potassium periodate, sodium periodate, perbromic acid, potassium perbromate, sodium perbromate and the like.

Of these oxidizing agents, particularly preferred are 3-iodoxybenzenecarboxylic acid, iodoxybenzene-3,4-dicarboxylic acid, 3-iodosobenzenecarboxylic acid, potassium iodate and sodium periodate. The oxidizing agent may be used preferably in an amount of from 1 mmol/l to 20 mmol/l, more preferably from 5 mmol/l to 15 mmol/l, as a concentration in an impregnating solution.

In the composition, combination of the buffer material, chromogen, stabilizing agent and oxidizing agent is not particularly limited, and each of them may be used as a single compound or as a mixture of two or more compounds.

The test strip of the present invention may be obtained by impregnating an absorbable carrier with one or more solution(s) or suspension(s) of the compounds described above together with necessary reagents for a desired measurement in a suitable solvent such as water, ethanol, methanol, acetone, etc., drying the impregnated carrier, and optionally adhering the resulting carrier to a support such as a plastic material or the like. When two or more impregnating solution(s) or suspension(s) are used, they may contain the compounds described above and necessary reagents for a desired measurement as a whole. Filter paper may be used most preferably as the absorbable carrier, but cotton, non-woven fabric, a piece of wood or the like may also be used. As an alternative means, the reagent-containing composition may be included in an organic polymer such as gelatin, a synthetic resin or the like which is subsequently applied to a support.

The thus obtained test strip may be used in the following manner. A test strip is dipped in a test sample such as urine and then taken out immediately. After a predetermined period of time, a color developed by the reaction of a peroxidatively active substance in the test sample with the chromogen in the test strip is compared macroscopically with a standard color scale which has been prepared in advance, thereby effecting qualitative and semi-quantitative measurement of the substance of interest in the sample. Also, it is possible to measure reflectance of the developed color using a meter and calculate concentration of the substance of interest from a calibration curve.

Though the stabilization mechanism and principle of the composition of the present invention are not clear yet, the inventive composition can be used markedly effectively for the redox reaction-based

measurement of peroxidatively active substances.

The following examples are provided to further illustrate the action and effect of the present invention. It is to be understood, however, that the examples are for purpose of illustration only and are not intended as a definition of the limits of the invention.

## EXAMPLE 1

Preparation of test strip for use in the measurement of urine hemoglobin:

In purified water were dissolved 1.5 mmol of nitrilotris(methylenephosphonic acid), 1 mmol of 3-iodoxybenzenecarboxylic acid, 1.5 g of cumene hydroperoxide, 7 g of β-cyclodextrin, 1 g of 2-aminobenzothiazole hydrochloride, 1 g of sodium lauryl sulfate, 50 ml of 1.0 M citrate buffer (pH 5), 100 mg of sodium ethylenediaminetetraacetate and 30 ml of ethanol, thereby obtaining a first impregnation solution (total volume, 100 ml).

A second impregnation solution was prepared by dissolving 1.5 g of o-tolidine in acetone (total volume, 100 ml).

A filter paper for chromatography use (3MM, Whatman Corp.) was impregnated with the first impregnation solution, dried at 60°C for 60 minutes, impregnated further with the second impregnating solution and again dried at 50°C for 20 minutes. Thereafter, the thus treated filter paper was cut into pieces of a predetermined size (5 x 5 mm), and each piece was adhered to a plastic support (5 x 80 mm) using a pressure sensitive adhesive double coated tape. In this way, a test strip for hemoglobin measurement use (to be referred to as "test strip A-1" hereinafter) was obtained.

As a control, a test strip (A-2) was prepared in the same manner except that nitrilotris(methylenephosphonic acid) was not used in the first impregnation solution.

## EXAMPLE 2

A test strip for hemoglobin measurement use (test strip B-1) was prepared by repeating the process of Example 1 except that nitrilodiacetic acid-methylenephosphonic acid was added to the first impregnation solution instead of nitrilotris(methylenephosphonic acid).

As a control, a test strip (B-2) was prepared in the same manner except that nitrilodiacetic acid-methylenephosphonic acid was not used in the first impregnation solution.

## EXAMPLE 3

A test strip for hemoglobin measurement use (test strip C-1) was prepared by repeating the process of Example 1 except that ethylenediaminetetrakis(methylenephosphonic acid) was added to the first impregnation solution instead of nitrilotris(methylenephosphonic acid).

As a control, a test strip (C-2) was prepared in the same manner except that ethylenediaminetetrakis(methylenephosphonic acid) was not used in the first impregnation solution.

## EXAMPLE 4

A test strip for hemoglobin measurement use (test strip D-1) was prepared by repeating the process of Example 1 except that cyclohexane-1,2-diaminetetrakis(methylenephosphonic acid) was added to the first impregnation solution instead of nitrilotris(methylenephosphonic acid).

As a control, a test strip (D-2) was prepared in the same manner except that cyclohexane-1,2-diaminetetrakis(methylenephosphonic acid) was not used in the first impregnation solution.

## EXAMPLE 5

A test strip for hemoglobin measurement use (test strip E-1) was prepared by repeating the process of Example 1 except that 3-iodosobenzenecarboxylic acid was added to the first impregnation solution instead of 3-iodoxybenzenecarboxylic acid.

As a control, a test strip (E-2) was prepared in the same manner except that nitrilotris(methylenephosphonic acid) was not used in the first impregnation solution.

EXAMPLE 6

A test strip for hemoglobin measurement use (test strip F-1) was prepared by repeating the process of Example 1 except that potassium iodate was added to the first impregnation solution instead of 3-iodoxybenzenecarboxylic acid.

As a control, a test strip (F-2) was prepared in the same manner except that nitrilotris-(methylenephosphonic acid) was not used in the first impregnation solution.

EXAMPLE 7

A test strip for hemoglobin measurement use (test strip G-1) was prepared by repeating the process of Example 1 except that sodium periodate was added to the first impregnation solution instead of 3-iodoxybenzenecarboxylic acid.

As a control, a test strip (G-2) was prepared in the same manner except that nitrilotris-(methylenephosphonic acid) was not used in the first impregnation solution.

EXAMPLE 8

A test strip for hemoglobin measurement use (test strip H-1) was prepared by repeating the process of Example 1 except that 3,3',5,5'-tetramethylbenzidine was added to the second impregnation solution instead of o-tolidine.

As a control, a test strip (H-2) was prepared in the same manner except that nitrilotris-(methylenephosphonic acid) was not used in the first impregnation solution.

EXAMPLE 9

A test strip for hemoglobin measurement use (test strip I-1) was prepared by repeating the process of Example 1 except that o-dianisidine was added to the second impregnation solution instead of o-tolidine.

As a control, a test strip (I-2) was prepared in the same manner except that nitrilotris-(methylenephosphonic acid) was not used in the first impregnation solution.

EXAMPLE 10

Performance comparison test -- 1 (ascorbic acid resistance):

Ascorbic acid resistances of test strips A-1 to I-1 prepared in Examples 1 to 9 were compared with that of a prior art test strip which has been prepared by repeating the process of Example 1 except that 3-iodoxybenzenecarboxylic acid was not used.

Test samples were prepared by adding 0, 25 or 50 mg/dl of ascorbic acid to normal human urine to which 0.06 mg/dl of hemoglobin has been added. Each of the test strips was dipped into each of the thus prepared test samples and taken out immediately. After 30 seconds the developed color was read by macroscopic observation using a standard color scale. The results are shown in Table 1.

TABLE 1

| Test strip | Concentration of ascorbic acid | | |
|---|---|---|---|
| | 0 mg/dl | 25 mg/dl | 50 mg/dl |
| Prior art strip | + | - | - |
| Test strip A-1 | + | + | ± to + |
| Test strip B-1 | + | + | ± to + |
| Test strip C-1 | + | + | ± to + |
| Test strip D-1 | + | + | ± to + |
| Test strip E-1 | + | + | ± to + |
| Test strip F-1 | + | + | ± to + |
| Test strip G-1 | + | + | ± to + |
| Test strip H-1 | + | + | ± to + |
| Test strip I-1 | + | + | ± to + |
| (In the Table, " + " means marked coloring, "±" means slight coloring, and "-" means negative coloring. These symboles in the following tables have the same meanings.) | | | |

As shown in the above table, the prior art test strip which contained a phosphonic acid analog of complexane but no oxidizing agent showed false negatives due to the effect of ascorbic acid. Contrary to this, each of the inventive test strips A-1 to I-1 to which an oxidizing agent for the reducing substance has been added did not undergo influences of the reducing substance (ascorbic acid) independent of the type of oxidizing agents used in the test strips (A-1, E-1, F-1, G-1). In addition, the ascorbic acid resistance did not change when different phosphonic acid analogs were used (A-1, B-1, C-1, D-1) or different chromogens were used (A-1, H-1, I-1). In this instance, the concentration of ascorbic acid was varied as shown in the table, because usually used urine samples contain ascorbic acid in an amount of 50 mg/dl or less in general.

EXAMPLE 11

Performance comparison test -- 2 (undesirable color development by added oxidizing agent):

Undesirable color development (false positive) caused by the oxidizing agents added to the test strips A-1 to I-1 prepared in Examples 1 to 9 was compared with that of the control test strips (A-2 to I-2) prepared in Examples 1 to 9. Hemoglobin-negative normal human urine was used as a test sample. Color development was checked in accordance with the aforementioned procedure, and the result was read by macroscopic observation using a standard color scale. The results are shown in Table 2.

TABLE 2

| A-1 | A-2 | B-1 | B-2 | C-1 | C-2 |
|---|---|---|---|---|---|
| - | ± | - | ± | - | ± |
| **D-1** | **D-2** | **E-1** | **E-2** | **F-1** | **F-2** |
| - | ± | - | ± | - | ± |
| **G-1** | **G-2** | **H-1** | **H-2** | **I-1** | **I-2** |
| - | ± | - | ± | - | ± |

As shown in the above table, the control test strips (A-2 to I-2) containing no stabilizing agent showed false positives due to the oxidation of chromogen caused by the added oxidizing agent for the reducing substance. Contrary to this, each of the inventive test strips (A-1 to I-1) containing a phosphonic acid analog as a stabilizing agent showed negative coloring.

EXAMPLE 12

Long-term storage stability:

Each of the inventive and control test strips A-1 and A-2 prepared in Example 1 was sealed in a vial in which a desiccating agent has been included. After 6 months of storage at room temperature, the test strips were used to measure of a hemoglobin-negative human normal urine sample, and the results were compared with those of the same but freshly prepared test strips. Test strips prepared in Examples 2 to 9 were also checked in the same manner. The results are shown in Table 3.

TABLE 3

| | A-1 | A-2 | B-1 | B-2 | C-1 | C-2 |
|---|---|---|---|---|---|---|
| Freshly prepared | - | ± | - | ± | - | ± |
| After 6 months | - | ± to + | - | ± to + | - | ± to + |
| | D-1 | D-2 | E-1 | E-2 | F-1 | F-2 |
| Freshly prepared | - | ± | - | ± | - | ± |
| After 6 months | - | ± to + | - | ± to + | - | ± to + |
| | G-1 | G-2 | H-1 | H-2 | I-1 | I-2 |
| Freshly prepared | - | ± | - | ± | - | ± |
| After 6 months | - | ± to + | - | ± to + | - | ± to + |

As shown in the above table, each of the test strips was stable for a prolonged period of time when a phosphonic acid analog was used as a stabilizing agent which was effective in preventing color development (false positive) caused by the addition of an oxidizing agent. In addition, unused test strips of Examples 1 to 9 (A-1 to I-1) showed no self-coloring in appearance even after 6 months of storage, while unused control test strips (A-2 to I-2) showed slight self-coloring in appearance after 6 months of storage. In consequence, the stabilizing agent can prevent undesirable coloring of test strips not only at the time of measurement (false positive) but also self-coloring in appearance after 6 months storage, thus improving long-term storage stability.

EXAMPLE 13

Humidity stability (self-coloring in appearance):

Each of the test strips A-1 and A-2 prepared in Example 1 was exposed to an atmosphere of 25°C in temperature and 60% in humidity. After 6 hours of exposure, coloring of each test strip in appearance was measured with the naked eye, and the result was compared with that of unexposed test strip. Test strips prepared in Examples 2 to 9 were also checked in the same manner. The results are shown in Table 4.

TABLE 4

| | A-1 | A-2 | B-1 | B-2 | C-1 | C-2 |
|---|---|---|---|---|---|---|
| Before exposure | - | - | - | - | - | - |
| After exposure | - | ± | - | ± | - | ± |
| | D-1 | D-2 | E-1 | E-2 | F-1 | F-2 |
| Before exposure | - | - | - | - | - | - |
| After exposure | - | ± | - | ± | - | ± |
| | G-1 | G-2 | H-1 | H-2 | I-1 | I-2 |
| Before exposure | - | - | - | - | - | - |
| After exposure | - | ± | - | ± | - | ± |

As shown in the above table, all of the control test strips containing no stabilizing agent showed self-coloring in appearance when exposed to a high humidity, while the inventive test strips showed improved humidity stability due to the use of the phosphonic acid analog as a stabilizer which was effective in preventing self-coloring in appearance.

EXAMPLE 14

Preparation of test strip for use in the measurement of urine glucose:

In purified water were dissolved 1.5 mmol of nitrilotris(methylenephosphonic acid), 1 mmol of 3-iodoxybenzenecarboxylic acid, 30 ml of 0.5 M citrate buffer (pH 6), 5,000 units of glucose oxidase, 5,000 units of peroxidase and 30 ml of ethanol, thereby obtaining a first impregnation solution (total volume, 100 ml).

A second impregnation solution was prepared by dissolving 1 g of 3,3',5,5'-tetramethylbenzidine, 1 g of polymethyl methacrylate and 1 g of polyvinyl formal in acetone (total volume, 100 ml).

Thereafter, a test strip for urine glucose measurement use (to be referred to as "test strip J-1" hereinafter) was obtained in accordance with the procedure of Example 1.

As a control, a test strip (J-2) was prepared in the same manner except that nitrilotris-(methylenephosphonic acid) was not used in the first impregnation solution. Another control test strip (J-3) was prepared in which 3-iodoxybenzenecarboxylic acid was not used in the first impregnation solution.

Example 15

Performance comparison of test strips for urine glucose measurement use:

The test strips prepared in Example 14 were checked for their performance. Firstly, test samples were prepared by adding 0, 25 or 50 mg/dl of ascorbic acid to normal human urine to which 100 mg/dl of glucose has been dissolved. Each of the thus prepared test samples was checked in accordance with the aforementioned procedure, and the result was read by macroscopic observation using a standard color scale to compare ascorbic acid resistances of the test strips. The results are shown in Table 5.

TABLE 5

| Test strip | Concentration of ascorbic acid | | |
|---|---|---|---|
| | 0 mg/dl | 25 mg/dl | 50 mg/dl |
| Test strip J-1 | + | + | ± to + |
| Test strip J-2 | + | + | ± to + |
| Test strip J-3 | + | - | - |

Next, undesirable colors (false positive) developed on each of the test strips J-1 to J-3 due to the added oxidizing agent were checked and compared. Glucose-negative normal human urine was used as a test sample, and the color development was read by macroscopic observation using a standard color scale. The results are shown in Table 6.

TABLE 6

| J-1 | J-2 | J-3 |
|---|---|---|
| - | ± | - |

As shown in the above tables, similar to the case of the urine hemoglobin measuring test strip, ascorbic acid resistance of the urine glucose measuring test strip was improved and undesirable coloring (false positive) of the strip due to the used oxidizing agent was prevented by the addition of a phosphonic acid analog as a stabilizing agent. In addition, similar to the case of the urine hemoglobin measuring test strip, excellent long-term storage stability and humidity stability were obtained (data not shown) and the urine glucose measuring test strip containing other stabilizing agent, oxidizing agent, chromogen and buffer

material exhibited good effects (data not shown).

When a reducing substance (ascorbic acid) is present in a test sample, peroxidatively active substances are consumed so that the test result is misjudged (false negative). Also, when a large amount of an oxidizing agent is used in order to avoid such an influence of the reducing substance, a chromogen contained in a test strip is oxidized and the test result is misjudged (false positive (±)) even in the case of a hemoglobin-negative sample or a glucose-negative sample. Such a negative sample can be judged correctly as negative when a phosphonic acid analog of complexane is included as a stabilizing agent in the test strip due to the effect of the analog to inhibit undesirable reaction of the chromogen with the oxidizing agent. In addition, the phosphonic acid analog can improve stabilities against various factors such as long-term storage and humidity, independent of the type of chromogen, oxidizing agent and the like.

The present invention provides a stable composition for use in the redox reaction-based measurement of peroxidatively active substances, which can be produced easily, is stable for a prolonged period of time and does not cause false negatives due to the presence of a reducing substance in a test sample or false positives due to the addition of an oxidizing agent in the composition, as well as a test strip comprising a support incorporated with the composition.

Although some preferred embodiments have been described, many modifications and variations may be made thereto in the light of the above teachings. It is to be understood therefore that within the scope of the appended claims, the invention may be practiced otherwise than as specifically described.

## Claims

1. A composition for use in the redox reaction-based measurement of peroxidatively active substances in a test sample, which comprises a buffer material, a chromogen in an amount sufficient to react with the peroxidatively active substance in a test sample to develop color, a phosphonic acid analog of complexane as a stabilizing agent in an amount sufficient to stabilize the composition, and an oxidizing agent in an amount sufficient to oxidize reducing substances in a test sample.

2. The composition according to claim 1 wherein said stabilizing agent is selected from the group consisting of phosphonic acid analog compounds of complexane represented by the following formulae I, II and III:

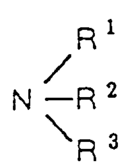

(I)

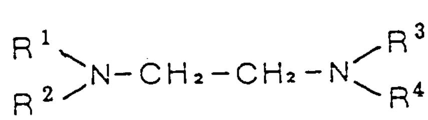

(II)

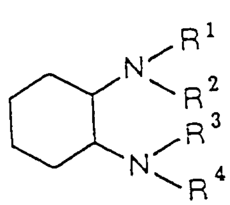

(III)

wherein $R^1$, $R^2$, $R^3$ and $R^4$ are the same or different groups, provided that at least one of the groups is an alkylenephosphonic acid group or a salt thereof, and the rest are selected from a hydrogen atom, a carboxyalkyl group and a hydroxyalkyl group.

3. The composition according to claim 2 wherein said stabilizing agent is selected from the group consisting of nitrilotris(methylenephosphonic acid), ethylenediaminetetrakis(methylenephosphonic acid), and cyclohexane-1,2-diaminetetrakis(methylenephosphonic acid).

**4.** The composition according to claim 1 or 2 wherein said oxidizing agent is selected from the group consisting of iodoxybenzene compounds represented by the following formula IV, iodosobenzene compounds represented by the following formula V and halogen oxyacid compounds represented by the following formulae VI and VII:

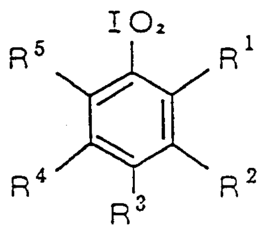

(IV) and

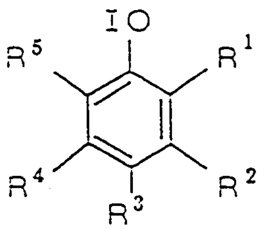

(V),

wherein $R^1$, $R^2$, $R^3$, $R^4$ and $R^5$ are the same or different groups selected from the class consisting of a hydrogen atom, a halogen atom, a carboxyl group, a sulfo group, a nitro group, a cyano group, a hydroxy group, an iodoxy group, a boro group, an arso group, a phospho group, and substituted or unsubstituted alkyl, alkoxy, aryl, arylsulfonyl, aryloxy, arylazo and amino groups; and

$MXO_3$ (VI) and

$MXO_4$ (VII)

wherein M is an atom or a group which is capable of becoming a monovalent cation, and X is a halogen atom.

**5.** The composition according to claim 4 wherein said oxidizing agent is selected from the group consisting of 3-iodoxybenzenecarboxylic acid, iodoxybenzene-3,4-dicarboxylic acid, 3-iodosobenzenecarboxylic acid, potassium iodate, and sodium periodate.

**6.** A test strip comprising a carrier incorporated with a composition for use in the redox reaction-based measurement of peroxidatively active substances in a test sample which comprises a buffer material, a chromogen in am amount sufficient to react with the peroxidatively active substance in a test sample to develop color, a phosphonic acid analog of complexane as a stabilizing agent in an amount sufficient to stabilize the composition, and an oxidizing agent in an amount sufficient to oxidize reducing substances in a test sample.

**7.** The test strip according to claim 6 which is obtainable by a process comprising steps of

preparing one or more impregnating solution(s) which contain a buffer material, a chromogen, a phosphonic acid analog of complexane as a stabilizing agent, and an oxidizing agent as a whole,

impregnating an absorbable carrier with said solution(s), and

drying the impregnated carrier.

**8.** The test strip according to claim 7 wherein said impregnating solution contains said phosphonic acid analog of complexane in an amount of from 5 mmol/l to 130 mmol/l.

**9.** The test strip according to claim 7 wherein said impregnating solution contains said phosphonic acid analog of complexane in an amount of from 10 mmol/l to 40 mmol/l.

**10.** The test strip according to claim 7 wherein said impregnating solution contains said oxidizing agent in an amount of from 1 mmol/l to 20 mmol/l.

**11.** The test strip according to claim 7 wherein said impregnating solution contains said oxidizing agent in an amount of from 5 mmol/l to 15 mmol/l.